# EUROPEAN PATENT APPLICATION

(11) **EP 3 991 796 A1**
(43) Date of publication of application: **04.05.2022**
(21) Application number: 20204062.2
(22) Date of filing: 27.10.2020
(51) Int. Cl.: A61P 35/04

(54) **AN EX VIVO METHOD FOR REMOVAL OF TUMOR CELLS FROM INTRA-OPERATIVELY SALVAGED BLOOD**

(71) Applicant: Lindis Blood Care GmbH, 16761 Henningsdorf (DE)
(72) Inventor: Lindhofer, Horst, 80639 München (DE); Bracht, Franzpeter, 13465 Berlin (DE); Heiss, Markus, 50968 Köln (DE)
(74) Representative: Isarpatent

(57) **Abstract**

The present invention relates to an *ex vivo* method for removal of tumor cells from intra-operatively salvaged blood which comprises contacting a trifunctional antibody with said blood to form aggregates or associates of antibody, tumor cells and immune cells, centrifuging to obtain an erythrocyte concentrate, and filtering to remove said aggregates or associates and residual antibody from said erythrocyte concentrate.

## Description

### Technical field

The present invention relates to a method of removing tumor cells from salvaged blood by using a trifunctional bispecific antibody and the reduction of residual antibody in the so produced erythrocyte concentrate.

### Background

Since the AIDS epidemic of the early 1980s the interest in alternatives to allogeneic blood transfusion has grown, particularly for elective surgery. One alternative that currently accounts for over 5 % of the blood donated in the United States and some countries in Europe is autologous transfusion, obtained primarily by preoperative donation. In addition to preoperative blood donation, intraoperative blood salvage (IBS) (Table A) from the surgical field represents an important option for covering transfusion demands. Along this IBS process, blood lost by a surgical patient is collected, cleaned, and made available for reinfusion to that patient.

Briefly, blood shed into the surgical field is aspirated from this site into an especially designed containment. Citrate or heparin anticoagulant is added, and the contents are centrifuged and/or filtered to remove leukocytes and clots and debris. IBS devices used can vary from simple, inexpensive, sterile bottles filled with anticoagulant to expensive, sophisticated, high speed cell washing devices (e.g. Medtronic Sequestra 1000, Cobe BRAT 2, Medtronic Autolog, Haemonetics Cell Saver-5^{®} and Fresenius CATS^{®}; Bentzien et al., Anaesthesist 49: 505, 2000; Serrick et al., J. Extra Corpor. Technol. 35(1): 28, 2003; Carless et al., The Cochrane Review, In: The Cochrane Library, John Wiley & Sons, Ltd., issue 3 pp. 1-180, 2010). Used in close to a million surgeries each year in the USA, the IBS procedure has become an integral part of blood management and conservation programs of hospitals (*www.bloodbook.com*).

Besides increased incidences of preoperative anemia, blood donation prior surgery raises serious additional economical questions due to the high overall transfusion rates (Carless et al., Transfus. Med. 14: 123, 2004). In addition, about 30 % of preoperative autologous blood donations have to be discarded in Italy because not-required autologous blood products are excluded from allogeneic blood transfusions *per se* according to regulatory guidelines. In this regard IBS and subsequent autotransfusion generally represent safe and more cost-effective measures in blood management.

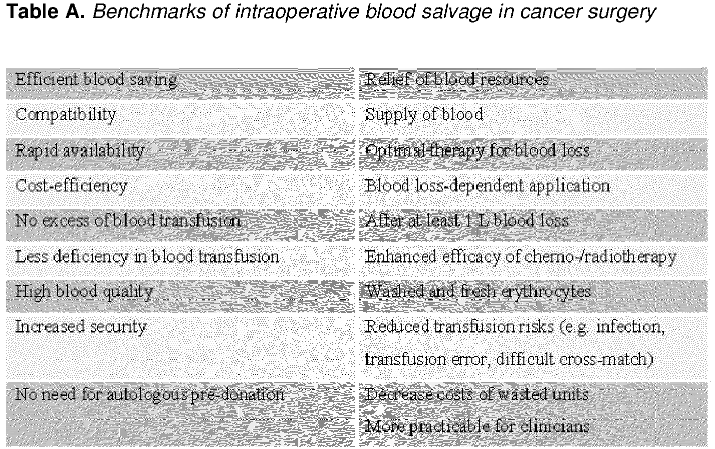

### Advantages of intraoperative blood salvage

In contrast to allogeneic red blood cell transfusions, IBS is considered a safe and efficacious alternative. Importantly, despite excellent viral diagnostics the risk of transferring HIV infection is 1 per 493.000 allogeneic blood transfusions, the risk of transferring hepatitis C virus infection is 1 per 103.000 and the risk of transmitting hepatitis B virus infection is 1 per 63.000 (Schreiber et al., N. Engl. J. Med. 334: 1685, 1996). Results of a 'Serious Hazards of Transfusions' study performed from 1996 until 2001 documented these risks of allogeneic transfusion incidences (Dzik et al., Transfusion 43: 1190, 2003). Strikingly, the transmission of infectious diseases by means of contaminated blood transfusions appears to be a minor risk compared to the enormous risks of ABO-incompatible blood due to administrative or human failure along the blood donation and transfusion process (Sazama, Transfusion 30: 583, 1990; Dzik et al., Transfusion 43: 1190, 2003). More than 70 % of transfusion incidences could be attributed to incorrect transfused blood components mainly caused by administration failure and errors in sampling, prescription as well as in component collection (Dzik et al., Transfusion 43: 1190, 2003). Pre- and intraoperative autotransfusion of red blood cells bypasses *per se* these inherent risks of allogeneic blood transfusion. In general, autologous blood salvage techniques offer advantages but do not require infusions of crystalloid or colloid to preserve blood volume (Table A). Many liters of blood can be salvaged intraoperatively during extensive bleeding, far more than with other autologous techniques.

### Suitability of patients for IBS

Intraoperative blood salvage has been available for over 25 years. It is used extensively in cardiothoriac surgery, vascular and trauma surgery, as well as liver transplantation. Contraindications to its use are bacterial infections and tumor cells probably shed into the blood of the surgery field, and use of microfibrillar collagen or other foreign material at the operative site. Nowadays, due to shortages of donor blood and fears of transmitted infections the use of IBS also gains great interest in cancer surgery with high blood loss. The reluctance of surgeons to use autotransfusion in cancer surgery has diminished, as reports found no increase in local recurrence or metastatic disease when compared with standard survival data (Klimberg et al., Arch. Surg. 212: 1326, 1986; Perseghin et al., Vox Sang. 72: 221, 1997). As reviewed by Vanderlinde et al. (BMJ 324: 772, 2002) red blood cell autotransfusions collected from preoperative blood donations could significantly lead to reduced infection and recurrence incidences during colorectal cancer surgery as compared to allogeneic transfusions (Table B). Some of these reviewed clinical trials are even more important as colorectal surgeries have been *per se* excluded from IBS recommendations due to the inherent risk of transmitting bacterial infections.

**Table B. Clinical outcome of randomized trials of autologous versus allogeneic transfusion***

| **Study** | **No of patients** | **Type of surgery** | **Intervention** | **Type of complication** | **% of cases developing complications after transfusion** | | **P value for reduction in postoperative complications** |
|---|---|---|---|---|---|---|---|
| | | | | | **Autologous** | **Allogeneic** | |
| Busch et al, 1993⁹ | 423 | Colorectal | Pre deposit autologous donation | Infection | 27 | 25 | NS |
| N. Engl. J. Med. 328: 1372, 1993 | | | | Recurrence | 37 | 34 | NS |
| Heiss et al, 1993 and 1994^{10, 14} | 120 | Colorectal | Predeposit autologous do nation | Infection | 12 | 27 | <0.05 |
| ***Lancet* 342:1328,1993** | | | | Recurrence | 17 | 29 | 0.11 |
| Newman et al. 1997¹¹ | 70 | Knee replacement | Postoperative autologous salvage | Infection | 6 | 34 | <0.05 |
| J. Bone Joint Surg. Br. 79: 630, 1997 | | | | | | | |
| Farrer et al, 1997¹² | 50 | Vascular | Intrao perative autologous salvage | Infection | 13 | 44 | 0.029 |
| J. Vasc. Nurs. 15:111, 1997 | | | | | | | |
| Thomas et al, 2001¹³ | 231 | Knee replacement | Postoperative autologous salvage | Infection | NA | NA | 0.036 |
| Br. J. Anaesth 86: 669, 2001 | | | | Readmission | NA | NA | 0.008 |

| | | | | | | | |
|---|---|---|---|---|---|---|---|
| NS=not significant, NA=not available (ie not reported). * Table is taken from Vanderlinde et al., BMJ 324: 772, 2002. | | | | | | | |

Nevertheless, a study clearly showed that tumor cells were detectable in the surgery field, although their impact on cancer recurrence remained unclear (Hansen et al., Arch. Surg. 130: 387, 1995). For instance, the number of tumor cells in the peripheral blood and IBS of 61 patients with cancer surgery of an abdominal, orthopedic, urological, gynecological, or head and neck malignant tumor were compared. In 57 of 61 patients, tumor cells were detected in the blood shed during oncological surgery. These tumor cells were identified by proliferation capacity, invasiveness, and tumorigenicity with a sensitivity of 10 tumor cells per 500 mL blood (Hansen et al., Arch. Surg. 130: 387, 1995). Interestingly, the number of tumor cells in the shed blood did not correlate with the amount of blood loss and only in 26 % of these patients circulating tumor cells could be detected in the peripheral blood. Therefore, it is estimated that the number of tumor cells in the blood shed into the surgery field could range from 10 to 10⁷. These results were also confirmed independently by Dale et al., Br. J. Surg. 75: 581, 1988 and Müller et al., Anaesthesist 45: 834, 1996.

To further address safety concerns about the risk of residual tumor cells in IBS samples, additional approaches are needed to effectively eliminate the contaminating tumor cells:
- One method used in combination with automatic IBS devices like Cell Saver-5^{®} is represented by additional filtration of samples through leukocytes depletion filters (e.g. Pall RC400, RCEZ1T), RC XL-1) (Bontadini et al., Transfusion 34. 531, 1994; Yaprak et al., Turk. J. Pediatr. 40: 89, 1998; Gwak et al., Liver Transplant. 11: 331, 2005). The safe transfusion of erythrocytes collected by IBS does not impair clinical outcome as evaluated in several trials (Edelman et al. Urology 47 : 179, 1996 ; Perseghin et al., Vox Sang. 72: 221, 1996; Davis et al., BJU International 91: 474, 2003).
- In a different IBS approach the samples are irradiated at 50 Gy due to the underlying principle of radiosensitivity of nucleated cancer cells and due to the radioresistance of non-nucleated red blood cells.

Due to the complex logistic demand of this IBS/irradiation approach including additional staff requirement, dosimetry issues, suitable as well as certified irradiation equipment in the clinical department, this latter procedure is not in favor of broad application. In contrast, the filtration procedure for leukocyte depletion represents an elegant approach to reduce residual tumor cells during IBS although this technique still has the inherent risk of residual tumor cells still passing the filter.

Therefore, for further enhanced safety in cancer surgery to remove residual tumor cells, all known methods for reintroducing autologous blood obtained e.g. during surgery from wounds of patients with tumors have to be improved in order to provide a reliable removal of possibly contaminating tumor cells from the salvaged blood; said improvement should be capable of being easily implemented in e.g. devices like Cell Saver-5^{®} or CATS^{®} or into any other methods to reintroduce blood from patients who had undergone surgery or during surgery.

WO-A-2013050445 described a method or removing tumor cells from salvaged blood, which is based on antibody-mediated formation of multi-cellular complexes, i.e. associates, comprising tumor cells recognized by antibody-driven tumor-associated antigen recognition, and optionally further by recognition of immune cells (leukocytes) like T cells and Fc-receptor positive accessory cells and/or further tumor cells followed by removal of said associates by e.g. centrifugation and/or filtration steps. Instead of or in combination with antibodies also protein scaffolds which mimic antibodies can be used. In this regard, however, there is a risk that the residual antibody is present in the treated blood in an excessive amount, whereby the reinfusion of said treated blood may cause severe side effects.

### Problems to be solved by the present Invention

The aim of the present invention is to provide an improved method of removing tumor cells from intra-operatively salvaged blood, which allows the reinfusion of autologous erythrocyte concentrates (EC) produced by cell saver device during oncological high blood loss surgery, wherein preferably the occurrence of side effects is also significantly decreased. In the present invention, it is revealed that the use of a trifunctional bispecific antibody which physically associates the tumor cells with immune cells, when combined with a step of centrifugation and a subsequent step of filtration, would result in EC with remarkably reduced tumor cells and residual antibody, and thereby said EC can be reinfused back into the patient without causing severe side effects.

### Detailed Description of the Invention and Preferred Embodiments

The following discussion is included for purposes of describing the present invention and illustrating preferred embodiments thereof.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of the present disclosure, suitable methods and materials are described below. The term "comprises" means "includes." All publications, patent applications, patents, and other references mentioned herein are incorporated by reference in their entirety. In case of conflict, the present specification, including explanations of terms, will control. In addition, the materials, methods, and examples are illustrative only and not intended to be limiting.

The present invention concerns:
(1) An ex vivo method for removal of tumor cells from intra-operatively salvaged blood comprising the following steps:
   (i) collecting an intra-operatively salvaged blood which may contain immune cells and tumor cells;
   (ii) contacting said intra-operatively salvaged blood from step (i) with at least one trifunctional antibody and/or with a scaffold protein, wherein said antibody and/or said scaffold protein comprises the following properties:
      a) binding to a T cell;
      b) binding to a tumor-associated antigen on a tumor cell;
      c) binding via its Fc-portion to an Fc-receptor positive cell,
      to obtain an intra-operatively salvaged blood containing cell aggregates, wherein said cell aggregates comprise said trifunctional antibody and/or said scaffold protein;
   (iii) separating an erythrocyte concentrate from the intra-operatively salvaged blood obtained from step (ii) via centrifugation, preferably by density gradient centrifugation;
   (iv) washing said erythrocyte concentrate from step (iii);
   (v) filtering the erythrocyte concentrate from step (iv) to remove residual of said aggregates and/or residual of said cell-bound trifunctional antibody and/or scaffold protein.
(2) Preferably, in the *ex vivo* method according to claim 1, said trifunctional antibody is selected from the group consisting of a bispecific, trispecific, tetraspecific and multispecific antibody, more preferably a whole IgG bispecific antibody.
(3) Preferably, in the *ex vivo* method according to (1) or (2), the amount of said at least one trifunctional antibody and/or said scaffold protein used to contact with said intra-operatively salvaged blood in step (ii) is 2.5 µg or more, more preferably 2.5 µg or more and 5.0 µg or less.
(4) Preferably, in the *ex vivo* method according to any one of (1) to (3), said intra-operatively salvaged blood in step (i) has a volume of 300 ml or more, preferably 400 ml or more before application of the trifunctional antibody. More preferably, said intra-operatively salvaged blood is collected in a reservoir.
(5) Preferably, the *ex vivo* method according to (4)further comprises a step of diluting the intra-operatively salvaged blood in step (i) to a volume of 350ml to 2800 ml, more preferably 350 ml to 2000 ml, which contains at least 300ml-500ml undiluted intraoperatively salvaged blood.
(6) Preferably, in the *ex vivo* method according to any one of (1) to (5), the method comprises at least one further round of steps (i) to (v), wherein at least in the first round the intra-operatively salvaged blood for contacting with said trifunctional antibody is in a volume of 400-1500 ml.
(7) Preferably, in the *ex vivo* method according to (6), the intra-operatively salvaged blood is a mixture of blood and dilution.
(8) Preferably, in the *ex vivo* method according to any one of (1) to (7), in step (ii) said at least one trifunctional antibody and/or said scaffold protein is contacted with said intra-operatively salvaged blood for a time period of 10 - 180 minutes to obtain the intra-operatively salvaged blood containing cell aggregates, preferably 20-90 minutes, more preferably 30-60 minutes, and optionally at a temperature of 19-25°C, preferably at room temperature.
(9) Preferably, in the *ex vivo* method according to any one of (1) to (8), said cell aggregates comprise said antibodies, tumor cells and immune cells, wherein the immune cells are preferably T cells and/or Fc-gamma receptor positive cells.
(10) Preferably, in the *ex vivo* method according to any one of (1) to (9), a filter is used in step (v) for filtering the erythrocyte concentrate from step (iv), and wherein said filter is preferably a leukocyte depletion filter.
(11) Preferably, in the *ex vivo* method according to any one of (1) to (10), said at least one trifunctional antibody is selected of a group of antibodies with the following isotype combinations:
   rat-IgG2b/mouse-IgG2a,
   rat-IgG2b/mouse-IgG2b,
   rat-IgG2b/human-IgG1,
   mouse-[VH-CH1; VL-CL]-human-IgG1/rat-[VH-CH1, VL-CL]-human-IgG1 -[hinge]-human-IgG3*-[CH2-CH3]
      [* = Caucasian allotypes G3m(b+g) = no binding to protein A].
(12) Preferably, in the *ex vivo* method according to any one of (1) to (11), said tumor associated antigen is selected from the group consisting of: EpCAM, Her2neu, EGFR, CD30, CD20, CD22, MUC1, MUC1* with changed glycosylation pattern, PSMA, CD33, MCSP, cMet, EphA2, Endosialin, Carboanhydrase IX, IGF-1R, FAP-alpha, CD19, GD2, CEA, FR, proteoglycans, G250, GC182, GT468, GT512, preferably the tumor associated antigen is EpCAM.
(13) Preferably, in the *ex vivo* method according to any one of (1) to (12), said trifunctional antibody and/or said scaffold protein binds to the T cell through a T cell surface antigen, and wherein the T cell surface antigen is selected from a group consisting of CD2, CD3, CD4, CD8, CD28, CD40L and CD44, preferably the T cell surface antigen is CD3.
(14) Preferably, in the *ex vivo* method according to any one of (1) to (13), said at least one trifunctional antibody and/or said scaffold protein comprises a binding site in its Fc-portion for Fcγ receptor type I, II and/or III.
(15) Preferably, in the ex vivo method according to any one of (1) to (13), said at least one trifunctional antibody and/or said scaffold protein is capable of binding monocytes, macrophages, dendritic cells, natural killer cells and/or activated neutrophils by their Fcγ receptor type I, II and/or III.
(16) Preferably, in the ex vivo method according to any one of (1) to (15), said tumor cells are from epithelial, hematological or neuroectodermal tumors.

Other preferred embodiments are described in the following description and the examples in combination with the Tables 1-3. Further preferred features of the invention can be taken from the claims.

The presently described method is practiced *ex vivo,* i.e. outside the human body. Intra-operative blood salvage (IBS) is well known in the art and is also described as "autologous blood salvage". Blood which is lost during surgery is recovered and re-infused into the same patient from whom the blood lost during surgery has been gained.

Additionally, the present invention relates to a trifunctional antibody which comprises the following properties:
a) binding to a T cell;
b) binding to a tumor-associated antigen on a tumor cell;
c) binding via its Fc-portion to an Fc-receptor positive cell,
   for use in a method of treating tumor or cancer, comprising (i) collecting an intra-operatively salvaged blood which may contain immune cells and tumor cells;
   (ii) contacting said intra-operatively salvaged blood from step (i) with said trifunctional antibody to obtain an intra-operatively salvaged blood containing cell aggregates, wherein said cell aggregates comprise said trifunctional antibody;
   (iii) separating an erythrocyte concentrate from the intra-operatively salvaged blood obtained from step (ii) via centrifugation, preferably by density gradient centrifugation;
   (iv) washing said erythrocyte concentrate from step (iii);
   (v) filtering the erythrocyte concentrate from step (iv) to remove residual of said aggregates and/or residual of said cell-bound trifunctional antibody.

Preferably, above-disclosed trifunctional antibody is selected from the group consisting of a bispecific, trispecific, tetraspecific and multispecific antibody, more preferably a bispecific antibody.

The method for removal of tumor cells from intra-operatively salvaged blood comprises the steps as described herein for the *ex vivo* method and what was said with regard to the *ex vivo* method applies accordingly to this method, where appropriate.

The presently described method is directed to the removal of tumor cells which potentially might contaminate intra-operatively obtained blood salvage. The blood is preferably obtained from patients who undergo a surgery treatment and suffer from a tumor and/or a cancer or who are suspected to harbor cells which might be considered to be tumorigenic.

Preferably, the method starts with the surgeon aspirating blood from the surgical field. The collecting step is preferably performed via a suction device and dilutives like e.g. sterofundin can also be used during this step. Other dilutives such as 0.9% NaCl with Heparin can also be used. The aspirated blood is then mixed with an anticoagulant in order to avoid coagulation of the blood. The aspirated blood may be collected in a reservoir until there is sufficient blood for processing.

Reference to an "anti-coagulant" includes classes of anti-coagulants such as anti-platelet drugs, thrombolytic and fibrinolytic drugs and metal ion chelators agents such as e.g. citrate, citrate dextrose (ACD) and EDTA and oxalate. In a related aspect, the anti-coagulants include heparin and glycosaminoglycans such as low molecular weight heparin such as Bemiparin, Certoparin, Dalteparin, Enoxaparin, Nadroparin, Pamaparin, Reviparin and Tinzaparin and heparinoid such as Danaparoid, Sulodexide, Dermatan sulfate; direct thrombin (II) inhibitors such as Argatroban, Bivalirudin, Dabigatran, Desirudin, Hirudin, Lepirudin, Melagatran, Ximelagatran; Fact. Xa inhibitors (such as Tick Anticoagulant Peptide), such as Apixaban, Otamixaban, Rivaroxaban and oligosaccharides such as Fondaparinux and Idraparinux; Vitamin K antagonists such as Acenocoumarol, Clorindione, Coumatetralyl, Dicoumarol (Dicumarol), Diphenadione, Ethyl biscoumacetate, Phenprocoumon, Phenindione, Tioclomarol and Warfarin.

In one embodiment of the present invention, the so collected blood salvage might be tested on the presence of tumor cells wherein particularly preferred the type of tumor-associated antigen is determined by well-known methods in the art, e.g. by labeled antibodies specifically reacting with epitopes of tumor-associated antigens in order to determine the type tumor-antigen to which said antibody binds and which is to be applied in the invention.

In the present invention, the term "tumor cell" refers to any cell which can divide relentlessly and form solid tumors or flood the blood with abnormal cells. Preferably, in the present invention, the tumor cells present in the intro-operatively salvaged blood can be from epithelial, haematological or neuroectodermal tumors.

Examples of tumors included in the present invention (but not limited thereto) comprise sarcomas and carcinomas, including fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyrgioma, ependymoma, pinealoma, hemangioblastoma, acoustic neuroma, oligodendroglioma, meningioma, melanoma, neuroblastoma and retinoblastoma). Further examples include epithelial tumors, hematological tumor and neuroectodermal tumors.

Preferably, in the *ex vivo* method of the present invention, said tumor cells are from epithelial, hematological or neuroectodermal tumors.

In the present invention, the "immune cells" include T cells and Fc-receptor positive cells. In the present invention, the "Fc-receptor cell" refers to cells with Fc-receptor present on the cell surface. Preferably, the "Fc-receptor positive cell" refer to one or more of monocyte, macrophage, dendritic cell, natural killer cell, neutrophil and eosinophilic cell.

Preferably, in step (i) the intra-operatively salvaged blood which may contain immune cells and tumor cells is collected in a reservoir.

The intra-operative blood salvage is then contacted with an antibody or an appropriate protein scaffold which is capable of specifically binding to at least one epitope of at least one tumor-associated antigen of at least one tumor cell.

In the present invention, "erythrocyte concentrate" refers to packed red cells.

In the present invention, "cell aggregate(s)" refers to cells which are adhered to each other, and said expression is interchangeable with "associate(s)" or "cell associate(s)". Similarly, "associating" and "aggregating" are also interchangeable.

As a further requirement, said antibody must be capable of forming a 3-dimensional network of at least said antibodies and said tumor cells contained in the blood in order to obtain aggregates or associates comprising said antibodies and tumor cells. In a further particularly preferred embodiment of the present invention, said antibody is also capable of binding to more than one tumor cells and/or to immune cells in order to form a three-dimensional network of antibodies and tumor cells and optionally immune cells and further optionally additional tumor cells. Due to the composition, structure and size of said associates resulting from the formation of multi-cellular complexes, the associates are capable of being removed from said IBS by centrifugation or filtration or a combination thereof. It is to be understood that filtration and centrifugation are preferred methods while other methods in order to efficiently remove said associates comprising residual tumor cells might be recognized by the person skilled in the art.

The invention therefore preferably focuses on the mechanical removal of associates and aggregates formed by associating and aggregating antibodies with tumor-associated antigens on tumor cells and immune cells, but not on the depletion of tumor cells carrying tumor-associated antigens by destruction of said tumor cells by antibody-specific interactions and immunological effects. While using antibodies in a conventional way as immunological agents in order to destroy tumor cells by interacting with immune cells, the invention gains benefit preferably on the antibodies' capacity of being capable in crosslinking antigens which are in the present case located on tumor cells or fragments thereof. The effects of the invention are achieved by the interaction of the antibodies with said tumor cells and particularly by removal of said three-dimensional network by centrifugal and filtration separation methods.

The present method can be also used in combination with other methods known in the art wherein antibodies or antibody-like molecules may be linked to magnetic components like magnetic beads or other structural elements which facilitate removal of antibody complexes via said structural elements, or tumor cells are removed by cell sorting methods like flow cytometry. Only through the cross-linking of tumor cells and immune cells by means of binding of the trifunctional antibody to tumor-associated antigens, CD3 on T cells and/or Fc-gamma receptor positive cells, via the Fc part of the AK, cell associates and aggregates can form. Due to said formation of a 3-dimensional network of antibodies with antigens like tumor-associated antigens on tumor cells or fragments thereof, and optionally immune cells and/or other tumor cells, a mechanical removal of said associates is possible. In this regard, in the present invention it is not necessary to involve the other structural components like magnetic beads or fluorescent molecules to facilitate said removal, which renders the present method more simple and easier to perform.

The present invention is herein described and claimed with respect to trifunctional bispecific antibodies within the limitations of the present invention, and exemplified with respect to the trifunctional bispecific antibody anti-CD3 x anti-EpCAM. Said anti-CD3 x anti-EpCAM trifunctional bispecific antibody is directed against tumor-associated antigen EpCAM, and is additionally binding to CD3, a T cell surface antigen and to Fc-receptor positive cells by its Fc-portion. The specific embodiments described in the examples have to be understood as exemplary embodiments which provide evidence for the feasibility of the present invention. Having provided evidence on the excellent removal of tumor cells from IBS by Catumaxomab, proof of concept has been given for the principle the presently claimed method is based upon. Having provided this evidence, the person skilled in the art will inevitably have the possibility to expand the concept to other tumors and other antibodies as far as covered by the present invention which are able to interact with said tumor cells and optionally said immune cells in order to provide for a three-dimensional network, i.e. multi-cellular complexes which can be removed for instance by centrifugation and/or filtration.

The antibodies of the present invention are specifically selected from trifunctional antibodies. Preferably, the trifunctional antibody in the present invention can be bi-, tri-, tetra- and multispecific antibodies. The trifunctional antibody disclosed below refers to trifunctional bispecific antibody. However, if the tri-, tetra- and multispecific antibodies also exhibit the same properties or effects, said trifunctional antibody can also refer to a trifunctional tri-, tetra- and multispecific antibody as used herein.

Generally, a bispecific antibody is defined as an antibody capable of binding to two different types of antigens preferably via its variable region; a trispecific antibody is characterized by binding to three different types of antigens preferably via its variable region; a tetraspecific antibody is characterized by binding to four different types of antigens preferably via its variable region while a multispecific antibody is defined as being capable of binding multiple different types of antigens preferably via its variable region. As one specific example, the trifunctional bispecific antibody anti-CD3 x anti-EpCAM is defined by binding to the tumor-associated antigen EpCAM on the one hand and to the T cell surface antigen CD3 on the other hand as well as with accessory cells by its Fc part.

Generally, the bi-, tri-, tetra- and multispecific antibodies described above may be monovalent, divalent, trivalent, tetravalent or multivalent. An antibody with a monovalent binding property is defined as an antibody which is capable of binding to one tumor-associated antigen. A bivalent monoclonal antibody is defined as an antibody which is capable of binding to two tumor-associated antigens or one tumor-associated antigen and one immune cell-associated antigen. A trivalent monoclonal antibody is defined as an antibody which is capable of binding to three different tumor-associated antigens or two tumor-associated antigens and one immune cell-associated antigen or one tumor-associated antigen and two immune cell-associated antigens. A tetravalent monoclonal antibody is defined as an antibody which is capable of binding to four different tumor-associated antigens or two different tumor-associated antigens - each having two identical antigen binding arms - or two/three tumor-associated antigens and one immune cell-associated antigen or two tumor-associated antigens and two immune cell-associated antigens. A multivalent monoclonal antibody is defined as an antibody which is capable of binding to one or more tumor-associated antigens and/or one or more immune cell associated antigen. The term "binding to a tumor-associated antigen" is defined as binding to an epitope of said tumor-associated antigen on a tumor cell. Only those antibodies having the trifunctional bispecific format as described by claim 1 are covered by the invention. All other antibodies are described only for information purposes.

General description of bifunctional or trifunctional antibodies are described by Kontermann RE (ed.), Springer Heidelberg Dordrecht London New York, pp. 1-28 (2011) having bispecific or trispecific (trifunctional formats with bivalent, trivalent and tetravalent) binding properties to one tumor-associated antigen and to one or more surface antigens of leukocytes (i.e. cells of the immune system) are of importance for this patent application.
- Bispecific antibody formats with bivalent antigen binding features:
   e.g. scFv (e.g. BiTE class), Db, scDb, dsDb, DART, dAb₂/VHH₂, knob-into-holes derivates, SEED-IgG, heteroFc-scfv, Fab-scFv, CrossMabs
- Bi- (tri-) specific antibody formats with trivalent antigen binding features:
   e.g. triple body, DNL-F(ab)3, scFv_{2-CH1/CL}, dAb₃, Fab-scFv₂, IgG-scFab
- Bi- (tri-) specific antibody formats with tetravalent antigen binding features:
   e.g. IgG-scFv, scFv-IgG, scFv-Fc, F(ab')_{2-SC}Fv₂, sDb-Fc, scDb-C_{H}3, Db-Fc, scFv₂-H/L, DVD-Ig, tandAb, scFv-dhlx-scFv, dAb₂-IgG, two-in-one mAb, mAb², dAb-IgG, dAb-Fc-dAb.

Additional antibodies to be used according to the invention are described in the following references:
Müller D and RE Kontermann. In: Bispecific Antibodies. Kontermann RE (ed.), Springer Heidelberg Dordrecht London New York, pp. 83-100 (2011)

### scFv (BiTE)

Baeuerle PA, Zugmaier G and D Rüttinger. In: Bispecific Antibodies. Kontermann RE (ed.), Springer Heidelberg Dordrecht London New York, pp. 273-288 (2011)

### DVD-Ig

Tarcsa E, Fraunhofer W, Ghayur T, Salfeld J and J Gu. In: Bispecific Antibodies. Kontermann RE (ed.), Springer Heidelberg Dordrecht London New York, pp. 171-186 (2011)

### DNL-derivatives

Chang C-H, Rossi EA, Sharkey RM, DM Goldenberg. In: Bispecific Antibodies. Kontermann RE (ed.), Springer Heidelberg Dordrecht London New York, pp. 199-216 (2011)

### Two-in-one antibodies

Koeing P and G Fuh. In: Bispecific Antibodies. Kontermann RE (ed.), Springer Heidelberg Dordrecht London New York, pp. 187-198 (2011)

### CrossMabs

Schaefer et al. Proc. Natl. Acad. Sci. USA 108: 11187 (2011)

In the present invention, at least one trifunctional antibody is used. Preferably, two or more trifunctional antibodies with different specificities can be combined for mediating the associates of tumor cells and immune cells.

Preferably, the intra-operatively salvaged blood in step (i) is undiluted with a volume of 300 ml or more, or, 400 ml or more. In this regard, "undiluted" means said intra-operatively salvaged blood is not mixed with any other liquid. More preferably, the intra-operatively salvaged blood in step (i) is undiluted with a volume of 2550 ml or less, 2000 ml or less, 1500 ml or less, or, 1300 ml or less. Even more preferably, the intra-operatively salvaged blood in step (i) is undiluted with a volume of 300 ml to 2550 ml, 350 ml to 2550 ml, 400 ml to 2550 ml, 300 ml to 2000 ml, 350 ml to 2000 ml, 400 ml to 2000 ml, 300 ml to 1500 ml, 350 ml to 1500 ml, 400 ml to 1500 ml, 300 ml to 1300 ml, 350 ml to 1300 ml, or 400 ml to 1300 ml.

Preferably, the antibody in step (ii) was applied only after a minimum collected intraoperative blood volume of 350ml in the reservoir to allow a better interaction and binding of the antibody with immune cells and tumor cells.

Preferably, the method in the present invention comprises a further step of diluting the intro-operatively salvaged blood in step (i) with a dilutive solution, wherein said dilutive solution is in a volume of 2500 ml or less, 2000 ml or less, 1500 ml or less, 1000 ml or less, 500 ml or less, 400 ml or less, 300 ml or less, 200 ml or less, or, 100 ml or less.

Preferably, the method in the present invention comprises a further step of diluting the intro-operatively salvaged blood in step (i) to a volume of 350 ml to 2800 ml, 350 ml to 2600 ml, 350 ml to 2400 ml, 350 ml to 2200 ml, 350 ml to 2000 ml, 400 ml to 2800 ml, 400 ml to 2600 ml, 400 ml to 2400 ml, 400 ml to 2200 ml, 400 ml to 2000 ml, 450 ml to 2800 ml, 450 ml to 2600 ml, 450 ml to 2400 ml, 450 ml to 2200 ml, 450 ml to 2000 ml, 500 ml to 2800 ml, 500 ml to 2600 ml, 500 ml to 2400 ml, 500 ml to 2200 ml, 500 ml to 2000 ml, 1000 ml to 2000 ml, 1000 ml to 2200 ml, 1000 ml to 2400 ml, 1000 ml to 2600 ml, 1200 ml to 2000 ml, 1200 ml to 2200 ml, 1200 ml to 2400 ml, 1200 ml to 2600 ml, 1400 ml to 2000 ml, 1400 ml to 2200 ml, 1400 ml to 2400 ml, or, 1400 ml to 2600 ml.

Preferably, the trifunctional antibody in the present invention is used in step (ii) in an amount of 1.0 µg or more, 1.5 µg or more, 2.0 µg or more, 2.5 µg or more, 3.0 µg or more, 3.5 µg or more, 4.0 µg or more, or, 4.5 µg or more. More preferably, the trifunctional bispecific antibody in the present invention in used in an amount of 2.5 µg or more. Even more preferably, the trifunctional bispecific antibody in the present invention is used in step (ii) in an amount of 1.0 µg to 1.5 µg, 1.5 µg to 2.0 µg, 2.0 µg to 2.5 µg, 2.5 µg to 3.0 µg, 3.0 µg to 3.5 µg, 3.5 µg to 4.0 µg, or, 4.0 µg to 4.5 µg. Preferably the concentration of the trifunctional antibody in the present invention is used in step (ii) in a concentration of 1 ng/ml to 7ng/ml, more preferably 2 ng/ml to 6 ng/ml, even more preferably 3 ng /ml to 5 ng/ml.

Preferably, the trifunctional antibody in the present invention is used in step (ii) in an amount of 5.0 µg or less. More preferably, the trifunctional bispecific antibody in the present invention is used in step (ii) in an amount of 1.0 µg or more to 5.0 µg or less, 1.5 µg or more to 5.0 µg or less, 2.0 µg or more to 5.0 µg or less, 2.5 µg or more to 5.0 µg or less, 3.0 µg or more to 5.0 µg or less, 3.5 µg or more to 5.0 µg or less, 4.0 µg or more to 5.0 µg or less, or, 4.5 µg or more to 5.0 µg or less. Even more preferably, the trifunctional bispecific antibody in the present invention is used in step (ii) in an amount of 2.5 µg or more to 5.0 µg or less.

Preferably, in the present invention, the steps (i) to (v) are repeated at least one time, and therein at least in the first round the intra-operatively salvaged blood for contacting with the trifunctional bispecific antibody is in a volume of 300 ml to 1500 ml, 350 ml to 1500 ml, 400 ml to 1500 ml, 450 ml to 1500 ml, or 500 ml to 1500 ml.

More preferably, the steps (i) to (v) are repeated at least one time, and therein at least in the first round the intra-operatively salvaged blood for contacting with the trifunctional bispecific antibody is in a volume of 300 ml to 1500 ml, 350 ml to 1500 ml, 400 ml to 1500 ml, 450 ml to 1500 ml, or 500 ml to 1500 ml, and wherein the intra-operatively salvaged blood is a mixture of blood and dilution.

The trifunctional antibody according to the present invention may bind to a T cell via a T cell surface antigen selected from a group consisting of CD2, CD3, CD4, CD8, CD28, CD40L and CD44. It means that the antibody for use according to the present invention preferably comprises a paratope which can recognize and bind to an epitope of a T cell surface antigen selected from the group consisting of CD2, CD3, CD4, CD8, CD28, CD40L and CD44. This specificity preferably promotes the recruitment of T cells.

Preferably, the T cell surface antigen is CD3. It means that the antibody for use according to the present invention further preferably comprises a paratope which can recognize and bind to an epitope of CD3.

Catumaxomab, an example for a trifunctional antibody, binds EpCAM-positive tumor cells and CD3-positive T-cells through its 2 specific binding sites. Catumaxomab also recruits FcyR type I, IIa and III-positive accessory cells via binding of its intact fragment crystallizable (Fc) region resulting in a trifunctional mode of action. The primary mode of action of Catumaxomab in the context of the present invention consists of the physical aggregation of tumor cells and lymphocytes/accessory cells and the subsequent removal of the cell aggregates by centrifugation and filtration. Beyond the mode of action of Catumaxomab when used in the present invention, several mechanisms of tumor cell destruction induced by bispecific trifunctional antibodies have been described.

Zeidler et al. [0], [0], [0] and Riechelmann et al. [0] elucidated the activation of T-cells and accessory cells in vitro, and their contribution to tumor cell killing, by BiUII (which is a variant antibody to Catumaxomab) and Catumaxomab. Using this trifunctional antibody with peripheral blood mononuclear cells (PBMCs), they showed that accessory cell activation is an important contributor to the antitumor activity. Activation of T-cells and accessory cells led to production of cytokines (interleukin [IL]-1β, IL-2, IL-6, IL-12, TNF-α, interferon-y [IFN-y] and Chemokine (C-C motif) ligand 18 [CCL18]). Remarkably, Riechelmann et al. demonstrated that IFN-γ reached peak values within 5 hours and TNF-α within 24 hours. They also demonstrated the upregulation of activation markers on dendritic cells and NK cells. Catumaxomab did not bind to B-lymphocytes, but it stimulated FcyR-positive accessory cells to eliminate tumor cells by direct phagocytosis.

In this context, especially for the present invention, the investigation of Arvå and Andersson [0] is of relevance showing that proinflammatory cytokines IL1β, TNF-α, IL-6, IL-8, IFN-γ and IL-12 were not secreted before 4 hours after stimulation. This time period is necessary after stimulation for de novo synthesis of cytokines which is the regular pathway [0].
[53] Zeidler R, Mayer A, Gires O, et al. TNF-alpha contributes to the anti-tumor activity of a bispecific, trifunctional antibody. Anticancer Res. 2001;21(5):3499-3503.
[54] Zeidler R, Mysliwietz J, Csanady M, et al. The Fc-region of a new class of intact bi-specific antibody mediates activation of accessory cells and NK cells and induces direct phagocytosis of tumor cells. Br J Cancer. 2000;83(2):261-266.
[55] Zeidler R, Reisbach G, Wollenberg B, et al. Simultaneous activation of T-cells and accessory cells by a new class of intact bi- specific antibody results in efficient tumor cell killing. J Immunol 1999;163(3):1246-1252.
[1] Riechelmann H, Wiesneth M, Schauwecker P, et al. Adoptive therapy of head and neck squamous cell carcinoma with antibody coated immune cells: a pilot clinical trial. Cancer Immunol Immunother. 2007;56(9):1397-1406.
[1] Abbas AK, Lichtman AH. General properties of cytokines. In: Cellular and Molecular Immunology. 5th edition. Philadephia, Pennsylvania: Saunders, imprint of Elsevier; 2003: Section IV.
[3] Arvå E, Andersson B. Kinetics of cytokine release and expression of lymphocyte cell-surface activation markers after in vitro stimulation of human peripheral blood mononuclear cells with Streptococcus pneumoniae. Scand J Immunol. 1999:49(3):237-243.

In a preferred embodiment, the antibodies used in the present invention are monoclonal antibodies. This is specifically true for the trifunctional bispecific antibodies disclosed herein in detail.

In the present invention, in case of higher volumes of intraoperative blood (e.g. >1500 ml), a new round of the here described procedure has to be started.

Therefore, preferably, after collection of 1500 ml intraoperative blood and supply with 2.5 µg or more antibody, more preferably 2.5 µg to 5.0 µg antibody, even more preferably 2.5 µg antibody, centrifugation has to be started first, before at least a further round of steps (i) to (v) can be started, wherein preferably 2.5 µg or more antibody, more preferably 2.5 µg to 5.0 µg antibody, even more preferably 2.5 µg antibody, is contacted with intraoperative blood, preferably in a volume of 1500 ml or less, in the further round(s) of steps (i) to (v).

Preferably, a minimal volume 300-400 ml of intraoperative blood must be collected in the IBS-reservoir before application of the antibody in the reservoir to avoid local over-concentration of antibody, whereby the advantage would be that a high local antibody concentration can be avoided, and a carry-over of excessive antibody levels into the final product EC can be significantly suppressed. Preferably, a minimum of undiluted intraoperative blood of 400 ml can be collected before starting the centrifugation to provide sufficient binding sites on lymphocytes for the antibody. Preferably, in the case that a maximum volume of 1500 ml collected intraoperative blood mixed with diluent is reached in the reservoir, a first centrifugation round of the IBS device can be started with the first antibody dose of 2.5 µg before, in case of further intraoperative blood collected from the surgical field, a further 2.5 µg dose of the antibody can be added to the reservoir.

Preferably, if more than 1500 ml of intraoperative blood is available from an individual patient, a second round of IBS can be performed if criteria, like for the first run are fulfilled. Additional runs e.g. foour or more, are preferably avoided to limit the amount of residual Ab being re-infused. Preferably, a total amount of residual antibody accumulated during two to three runs in the ECs should not be more than 70 ng.

Proteins having relatively defined three-dimensional structures are commonly referred to as protein scaffolds. These protein scaffolds may be used as reagents for the design of artificially engineered antibodies. These scaffolds typically contain one or more regions which are amenable to specific or random sequence variation, and such sequence randomization is often carried out to produce libraries of proteins from which the desired antibody scaffolds may be selected. Such scaffolds are particularly useful in the field of antibody design.

These antibody scaffolds are non-immunoglobulin proteins which mimic properties of a monoclonal antibody with respect to its binding activity to for instance tumor cells and immune cells. Scaffolds often include loops or domains which form the binding side of said antibody scaffold. These antibody mimics may be utilized for the purpose of designing proteins which are capable of binding to virtually any compound of interest. This directed evolution approach results in the production of antibody-like molecules with high affinities for antigens of interest. In addition, those scaffolds may be used to display defined exposed loops (e.g. loops previously randomized and selected on the basis of antigen binding) in order to direct evolution of molecules that bind to such introduced loops. Methods on how to obtain antibody-like scaffold proteins are known in the art. The following describes one possible approach for obtaining an antibody-like scaffold protein.

A first screening method, useful for the isolation or identification of randomized or mutated proteins of interest, involves: (a) contacting a compound of interest with a candidate protein, the candidate protein being a derivative non-antibody protein including a domain having an immunoglobulin-like fold, the non-antibody protein deriving from a reference protein by having a mutated amino acid sequence wherein the non-antibody protein binds with a Kd at least as tight as 1 microM to a compound that is not bound as tightly by the reference protein, wherein the contacting is carried out under conditions that allow compound-protein complex formation; and (b) obtaining, from the complex, the derivative protein that binds to the compound.

The second screening method is for isolating or identifying a compound which binds to a tumor-associated protein of interest. This method begins with a non-antibody protein including a domain having an immunoglobulin-like fold and deriving from a reference protein by having a mutated amino acid sequence, wherein the non-antibody protein binds with a Kd at least as tight as 1 µM to a compound that is not bound as tightly by the reference protein. This derivative protein is then contacted with a candidate compound (tumor-associated antigen or an epitope thereof), wherein the contacting is carried out under conditions that allow compound-protein complex formation, and the compound which binds to the derivative protein is obtained from the complex. Again, this general technique may be carried out with any protein.

Further methods of obtaining non-antibody proteins which bind to compounds of interest (tumor-associated antigen or an epitope thereof) are described as follows. One such method involves: (a) providing a non-antibody scaffold protein including an immunoglobulin-like fold, wherein the scaffold protein does not bind to the compound with a Kd as tight as 1 micro M; (b) generating mutated derivatives of the non-antibody scaffold protein, thereby producing a library of mutated proteins; (c) contacting the library with the compound; (d) selecting from the library at least one derivative protein which binds to the compound with a Kd at least as tight as 1 µM; and (e) optionally repeating steps (b)- (d) substituting for the non-antibody scaffold protein in repeated step (b) the product from the previous step (d). Again, this general technique may be carried out with any protein.

The so produced scaffold proteins mimic the function of an antibody as disclosed above and below and can be used either instead of an immunoglobulin-based antibody or in combination with it. In the present invention, said trifunctional antibody can be replaced with said scaffold protein insofar both display the same function in respect of binding to a T cell, binding to a tumor-associated antigen on a tumor cell, and binding via its Fc-portion to an Fc-receptor positive cell, to obtain an intra-operatively salvaged blood containing cell aggregates. In the present invention, said trifunctional antibody and said scaffold protein can be applied alone or in combination.

The following describes in more detail trifunctional bispecific and trispecific antibodies. It is to be noted that the trifunctional bispecific and trispecific antibodies are herein described also with regard to their mode of action in the human body in order to provide for a full definition of these monoclonal antibodies. However, these intrinsic properties of said trifunctional bispecific and trispecific antibodies have no effect in the presently claimed invention as the antibodies are removed from the IBS almost completely, at least up to a non-detectable or non-relevant extent so that they do not provide any relevant mode of action in the human body after reinfusion of the tumor cell- depleted erythrocyte concentrate. Specifically, the presently described method is performed *in vitro* outside the human body, which can be illustrated by the above-stated Catumaxomab binding with EpCAM-positive tumor cells and CD3-positive T-cells through its 2 specific binding sites and the removal thereof, so that any intrinsic biological activity of the antibodies cannot occur.

The antibodies to be used in the present invention are preferably characterized by the additional effects of binding of the Fc receptor-positive cell by binding to the Fc receptor-positive cell via Fcγ receptors of type I, II and III.

Preferably, the antibody used according to the invention comprises a binding site in its Fc-portion for Fcγ receptor type I, II and/or III.

Preferably, the antibody used according to the invention is able to bind to monocytes, macrophages, dendritic cells, natural killer cells, neutrophils and/or eosinophilic cells being Fcγ receptor positive cells.

In the present invention, the tumor associated antigen refers to an antigenic substance produced in tumor cells which is expressed on the surface of a tumor cell.

Preferably, the tumor associated antigen the trifunctional antibody or scaffold protein binds to is selected from the group consisting of: EpCAM, Her2neu, EGFR, CD30, CD20, CD22, MUC1, MUC1* with changed glycosylation pattern, PSMA, CD33, MCSP, cMet, EphA2, Endosialin, Carboanhydrase, IGF-1R, FAP-alpha, CD19, GD2, CEA, FR, proteoglycans, G250, GC182, GT468, and GT512.

More preferably, said tumor associated antigen the trifunctional antibody or scaffold protein binds to is EpCAM, Her2/neu, MUC1, EGFR, EphA2, GD2 or CD20.

Preferably, the trifunctional antibody of the present invention is directed against one T cell surface antigen and one tumor-associated antigen, wherein the T cell surface antigen which is selected from a group consisting of CD2, CD3, CD4, CD8, CD28, CD40L and CD44, can be combined with any tumor-associated antigen selected from a group consisting of EpCAM, Her2neu, EGFR, CD30, CD20, CD22, MUC1, MUC1* with changed glycosylation pattern, PSMA, CD33, MCSP, cMet, EphA2, Endosialin, Carboanhydrase, IGF-1R, FAP-alpha, CD19, GD2, CEA, FR, proteoglycans, G250, GC182, GT468, and GT512.

Preferably, the trifunctional antibody of the present invention is directed against one T cell surface antigen which is CD3 and one tumor-associated antigen which is EpCAM.

Preferred antibodies are heterologous trifunctional bispecific antibodies, preferably monoclonal, selected from one or more of the following combinations of isotypes:
- rat-IgG2b/mouse-IgG2a,
- rat-IgG2b/mouse-IgG2b,
- rat-IgG2b/mouse-IgG3;
- rat-IgG2b/human-IgG1,
- rat-IgG2b/human-IgG2
- rat-IgG2b/human-IgG3 [oriental allotype G3m(st)=binding to protein A], rat-IgG2b/human-IgG4;
- rat-IgG2b/rat-IgG2c;
- mouse-IgG2a/human-IgG3 [caucasian allotypes G3m(b+g)=no binding to protein A, in the following indicated as *]
- mouse-IgG2a/mouse-[VH-CH1, VL-CL]-human-IgG1-[hinge]-human-IgG3*-[CH2-CH3]
- mouse-IgG2a/rat-[VH-CH1, VL-CL]-human-IgG-[hinge]-human-IgG3*-[CH2-CH3]
- mouse-IgG2a/human-[VH-CH1, VL-CL]-human-IgG-[hinge]-human-IgG3*-[CH2-CH3]
- mouse-[VH-CH1, VL-CL]-human-IgG1/rat-[VH-CH1, VL-CL]-human-IgG1-[hinge]-human-IgG3*-[CH2-CH3]
- mouse-[VH-CH1, VL-CL]-human-IgG4/rat-[VH-CH1, VL-CL]-human-IgG4-[hinge]-human-IgG4 [N-terminal region of CH2]-human-IgG3*[C-terminal region of CH2:>aa position 251]-human-IgG3*[CH3]
- at-IgG2b/mouse-[VH-CH1, VL-CL]-human-IgG1-[hinge-CH2-CH3]
- rat-IgG2b/mouse-[VH-CH1, VL-CL]-human-IgG2-[hinge-CH2-CH3]
- rat-IgG2b/mouse-[VH-CH1, VL-CL]-human-IG3-[hinge-CH2-CH3, oriental allotype]
- rat-IgG2b/mouse-[VH-CH1, VL-CL]-human-IgG4-[hinge-CH2-CH3]
- human-IgGl/human-[VH-CH1, VL-CL]-human-IgG-[hinge]-human-IgG3*-[CH2-CH3]
- human-IgG1/rat-[VH-CH1, VL-CL]-human-IgG1-[hinge]-human-IgG4 [N-terminal region of CH2]-human-IgG3*[C-terminal region of CH2:>aa position 251]-human-IgG3*[CH3]
- human-IgG1/mouse-[VH-CH1, VL-CL]-human-IgG1-[hinge]-human-IgG4 [N-terminal region of CH2]-human-IgG3*[C-terminal region of CH2:>aa position 251]-human-IgG3*[CH3]
- human-IgG1/rat-[VH-CH1, VL-CL]-human-IgG1-[hinge]-human-IgG2 [N-terminal region of CH2]-human-IgG3*[C-terminal region of CH2:>aa position 251]-human-IgG3*[CH3] human-IgG1/mouse-[VH-CH1, VL-CL]-human-IgG1-[hinge]-human-IgG2 [N-terminal region of CH2]-human-IgG3*[C-terminal region of CH2:>aa position 251]-human-IgG3*[CH3]
- human-IgG1/rat-[VH-CH1, VL-CL]-human-IgG-[hinge]-human-IgG3*-[CH2-CH3]
- human-IgG1/mouse-[VH-CH1, VL-CL]-human-IgG-[hinge]-human-IgG3*-[CH2-CH3]
- human-IgG2/human-[VH-CH1, VL-CL]-human-IgG2-[hinge]-human-IgG3*-[CH2-CH3]
- human-IgG4/human-[VH-CH1, VL-CL]-human-IgG4-[hinge]-human-IgG3*-[CH2-CH3]
- human-IgG4/human-[VH-CH1, VL-CL]-human-IgG4-[hinge]-human-IgG4 [N-terminal region of CH2]-human-IgG3*[C-terminal region of CH2:>aa position 251]-human-IgG3*[CH3]
- mouse-IgG2b/rat-[VH-CH1, VL-CL]-human-IgG-[hinge]-human-IgG3*-[CH2-CH3]
- mouse-IgG2b/human-[VH-CH1, VL-CL]-human-IgG1-[hinge]-human-IgG3*-[CH2-CH3]
- mouse-IgG2b/mouse-[VH-CH1, VL-CL]-human-IgG1-[hinge]-human-IgG3*-[CH2-CH3]

The trifunctional bispecific antibody with monovalent binding specificities used in one particularly preferred embodiment by the present invention has the following properties:
a) binding to a T cell;
b) binding to a tumor-associated antigen on a tumor cell;
c) binding via its Fc-portion to an Fc-receptor positive cell;
   the trifunctional bispecific antibody is further preferably selected of a group of antibodies with the following isotype combinations:
   rat-IgG2b/mouse-IgG2a,
   rat-IgG2b/mouse-IgG2b,
   rat-IgG2b/human-IgG1,
   mouse-[VH-CH1; VL-CL]-human-IgG1/rat-[VH-CH1, VL-CL]-human-IgG1-[hinge]-human-IgG3*-[CH2-CH3]
   [* = Caucasian allotypes G3m(b+g) = no binding to protein A].

Specifically preferred is an antibody, preferably a trifunctional bispecific antibody and/or a scaffold protein directed against EpCAM and CD3 with the isotype combination rat-IgG2b/mouse-IgG2a. A preferred example for said trifunctional bispecific antibody is anti-CD3 x anti-EpCAM antibody. Preferably said antibody is monoclonal.

Preferably, the antibodies according to the invention are monoclonal, chimeric, recombinant, synthetic, semi-synthetic, or chemically modified intact antibodies having for example Fv, Fab, scFv, or F (ab)2 fragments.

In the method of the present invention also antibodies or derivatives or fragments of human origin can be used, or antibodies modified to be suitable for the use in humans (so-called "humanized antibodies") (see for example Shalaby et al., J. Exp. Med. 175 (1992), 217; Mocikat et al., Transplantation 57 (1994), 405).

The preparation of the different types of antibodies and antibody fragments mentioned above is obvious to the skilled artisan. The preparation of monoclonal antibodies preferably of mammalian origin, e.g. of human, rat, mouse, rabbit, or goat, can be performed using conventional methods for example as those described in Köhler and Milstein (Nature 256 (1975), 495), in Harlow and Lane (Antibodies, A Laboratory Manual (1988), Cold Spring Harbor) or in Galfre (Meth. Enzymol. 73 (1981), 3).

It is further possible to prepare the antibodies described by means of recombinant DNA technology according to techniques obvious to the skilled artisan (see Kurucz et al., J. Immunol. 154 (1995), 4576; Hollinger et al., Proc. Natl. Acad. Sci. USA 90 (1993), 6444).

The preparation of antibodies having two different specificities, the so-called bispecific antibodies, can be performed for example using recombinant DNA technology but also by the so-called hybrid hybridoma fusion technique (see for example Milstein et al., Nature 305 (1983), 537). This technique comprises fusing hybridoma cell lines each producing antibodies having one of the desired specificities and identifying and isolating recombinant cell lines producing antibodies having both specificities.

The problem forming the basis of the invention can be overcome by using in preferred embodiments either trifunctional bispecific or trispecific trifunctional antibodies if they exhibit the properties and effects as described herein. The invention is particularly described by the way of trifunctional bispecific antibodies. However, it is understood that it also covers the following trispecific antibodies exhibiting similar effects. Although above, the terms "antibody" or "scaffold protein" may refer to trifunctional bispecific antibody, it is understood that it can also covers the following trispecific antibodies exhibiting similar effects.

The preparation of antibodies exhibiting three specificities, so-called trispecific antibodies, also suitable to solve the problem of the invention, may for example be carried out by coupling a third antigen binding site having an additional specificity, e.g. in the form of "single chain variable fragments" (scFv) to one of the IgG heavy chains of a bispecific antibody. Further, recombinant technology can be used, e.g. vector-based methods for protein synthesis or oligonucleotide synthesis.

Analogously, trispecific F(ab)2 constructs may be prepared by replacing the CH2-CH3 regions of the heavy chain of one specificity of a bispecific antibody by an scFv having a third specificity, while the CH2-CH3 regions of the heavy chain having the other specificity can be removed for example by insertion of a stop codon (at the end of the "hinge" 5 region) into the coding gene, e.g by homologous recombination.

It is also possible to prepare trispecific scFv constructs wherein three VH-VL regions representing three different specificities are arranged in series.

Intact bispecific antibodies are composed of two antibody semi-molecules (each having a H and a L immunoglobulin chain) each representing a specificity, and additionally like normal antibodies having a Fc portion performing the well-known effector functions. They are preferably prepared using the quadroma technology. This method of preparation is exemplified in DE-A-44 19 399. For complete disclosure this document is incorporated in its entirety by reference also with respect to a definition of bispecific antibodies. It should be understood that other methods of preparation are also useful if they lead to the intact bispecific antibodies according to the above definition required according to the invention.

For example, intact bispecific antibodies may be produced in sufficient amounts using a newly developed method of preparation (Lindhofer et al., J. Immunology, 155:219 (1995)). The combination of two bispecific antibodies directed against two different tumor-associated antigens (e.g. c-erb-B2, EpCAM, such as GA-733-2=C215) on the mammary carcinoma cells minimizes the risk that tumor cells expressing only one of the antigens remain unidentified.

In accordance with the method of the present invention, the antibodies or the scaffold proteins recognizing the tumor-associated antigens on a tumor cell are not bound to or by any means like magnetic particles or magnetic beads which might have been used in the art as a tool in order to remove associates which are formed via the binding of said antibodies to said tumor cells and optionally also immune cells. Further, the prior art describes the labeling of the tumor cells or the antibodies with e.g. chromogenic substances like fluorochromes followed by cell sorting like flow cytometry. Contrary thereto, the principle of the invention lies in the formation of three dimensional networks (associates) as described above of a size sufficient to be retained by filters or to be separated by centrifugal forces and the direct removal of said networks by mechanical means like filtration or centrifugation or a combination thereof. The size of the associates formed by interaction with the antibodies is in a magnitude to be removable by filtration or centrifugation. Embodiments with magnetic beads or similar tools which bind to the antibodies or the tumor cells as indirect auxiliary means to remove the associates between the antibodies and the tumor cells can be combined with the present invention. Also separation methods like flow cytometric methods wherein tumor cells are separated from normal cells can also be combined with the present invention.

The terms "associates", "multi-cellular complexes" and "aggregates" are used interchangeably and always define a three-dimensional network between antibodies, scaffold proteins, tumor cells and/or immune cells and/or further tumor cells in order to form cross-linked tumor cells which are removable by centrifugation or filtration or a combination thereof. Said associates are specifically comprised of antibodies, tumor cells and immune cells which are T cells and/or Fc-receptor positive cells.

The at least one antibody is contacted with said intra-operatively obtained blood salvage for a time period which is sufficient to cross-link tumor cells and optionally immune cells and/or other tumor cells in order to obtain associates. Preferably, said tumor cells and said immune cells are present in said intra-operatively obtained blood salvage. Said tumor cells can be specifically recognized by said at least one antibody or said at least one scaffold protein.

The time period which is necessary to achieve said cross-linkage can be determined by a person skilled in the art with routine methods. The time period used in the present invention is preferably 10 to 180 minutes to obtain the intra-operatively salvaged blood containing cell aggregates. In the present invention, a contact period is preferably between 10 and 118 minutes, more preferably between 20 and 90 minutes and even more preferably between 30 and 60 minutes. The temperature is preferably room temperature which might range from 19°C to 25°C, preferably about 21°C.

The filtration is preferably a leukocyte reduction or depletion filtration wherein aggregates or associates containing tumor cells are all or substantially removed while the erythrocytes pass the filter and are collected. The filters might be selected from screen filters with pore sizes of between 20 µm to 40 µm which retain the associates formed by the method of the invention and also for instance fibrin strands and clumps of dead cells. Erythrocytes which are about 8 µm in size may pass through the filters. Micro aggregate filters are preferably used for the re-infusion of shed autologous blood collected during or after surgery.

A review describing the cell saver devices including separation methods is Carless PA, Henry DA, Moxey AJ, O'Connell D, Brown T, Fergusson DA, Cell salvage for minimizing perioperative allogeneic blood transfusion, 2010, The Cochrane Collaboration, John Wiley & Sons, Ltd. which is fully incorporated by reference.

An example for a micro-aggregate blood filter in order to receive an erythrocyte containing fraction only is Pall SQ 40S Blood Transfusion Filter.

Centrifugation is performed generally by a density gradient centrifugation step followed by washing with physiological saline for a time and with a rotational speed sufficient to remove said tumor cells-comprising associates and to separate erythrocytes from said tumor cell associates and optionally from leukocytes.

Filtration is performed generally for a time period sufficient to remove said tumor cells-comprising associates and to separate erythrocytes from said tumor cell associates and optionally from leukocytes.

In a preferred embodiment said concentrate of erythrocytes which is free or substantially free of contaminating tumor cells is used without addition of physiological saline.

Further filtration steps may be used in order to remove residual associates and/or leukocytes.

As the underlying filtration technology is different from classical, micron-rated filtration procedures including clogging issues, this advanced tumor cell removal strategy appears to be feasible.

It is one of the benefits of the invention is that the time involved for said removal of tumor cells is dramatically reduced by the invention compared to the depletion of tumor cells by destruction with antibodies or by removal with magnetic beads or cell sorting methods.

Other advantages of the present invention include that tumor cells from the produced erythrocyte concentrate out of intraoperative blood are remarkably reduced, and proinflammatory cytokines produced in the operational field by traumata through surgery is also significantly decreased. Moreover, residual trifunctional antibody can be reduced to uncritical amount in the final EC concentrate ready for reinfusion.

The blood products obtained by using the method of the invention are - according to a preferred embodiment - then re-administered into the patient from whom the starting blood sample was obtained, which would not cause severe side effects.

### Example

In the following, one exemplary example for carrying out the invention is described. A person with ordinary skill in the art following this approach will inevitably result in obtaining the claimed benefit. Following this example, various modifications can be performed without deviating from the invention. The following Example is merely for illustrating the above disclosure, and should not be seen as limiting the scope of present invention.

In the preparatory *in vitro* study, three parameters were investigated in intraoperative blood, during processing and in the final product to demonstrate safety and efficacy:
1) EpCAM positive tumor cells
2) Proinflammatory cytokines and
3) Residual antibody Catumaxomab.

The analysis of the tumor cells demonstrated their presence in 63% of intraoperative blood samples ranging from 69 up to about 260.000 within 10 of 16 tested patient samples. Importantly no residual tumor cells were detected in the final erythrocyte concentrates produced during the Catuvab procedure, i.e. Using a cell saver device (also called mechanically processed autotransfusion machine (MAT)) including the application of a trifunctional bispecific antibody to generate cell aggregates by crosslinking immune cells with tumor cells and combining centrifugation and filtration steps to produce a tumor cell free erythrocyte concentrate.

To cover the safety aspect of the Catuvab procedure, proinflammatory cytokines IL-6 and IL-8 were measured representing two of the most relevant indicators for potential side effects after reinfusion of ECs. A 28-fold reduction in mean was shown for the total amounts of IL-6 in the final product (EC) compared to the unprocessed collected intraoperative blood in the reservoir. For IL-8 even a 52-fold reduction in mean was observed. The mean values of the total amounts for IL-6 and IL-8 in the final EC product were 53 and 9ng, respectively representing uncritical values having in mind that these amounts would be diluted in the patient's body about 2000-3000 fold. This interpretation is based on the calculation that patients body blood volume ranges in average between 5-7 liters containing about 2-3 liters of plasma. Even the measured peak value of 264ng of IL-6 would not lead to critical values in this scenario. Thus, from the data of this study, safety aspects influenced by proinflammatory cytokines should not be an issue for the Catuvab procedure.

The third important parameter especially in light of an approval of the Catuvab procedure as medical device is the amount of residual antibody in the final EC product.

Here, two changes of the procedure were introduced during the study (described in the results section) which led to strongly reduced residual antibody in the final product. Thus, after implementation of the changes only total antibody amounts between the quantification limit (125pg/ml) and 9ng were found in the final EC product. A recommendation can be given based on the measured values of all 15 patients which received antibody, where the peak value was 69ng in the final product. Therefore, an amount of 70ng antibody in the final EC product could be a value used to define a limit for residual antibody.

The purpose of the study was to utilize Catuvab for the removal of tumor cells from patient blood collected during oncological surgery and to assess tumor cells, cytokines and residual Catumaxomab antibody before and during the Catuvab -procedure and in the final product, the erythrocyte concentrate (EC).

The Catuvab procedure uses (i) the bispecific trifuntional antibody Catumaxomab to crosslink EpCAM positive tumor cells with CD3 positive T-cells and Fc-gamma receptor positive immune cells and (ii) to remove such cell aggregates during a centrifugation step from the so generated EC and (iii) to remove residual tumor cell containing cell aggregates during a final filtration step using a 40µm leukocyte depletion filter (LDF).

### 1. Material

### 1.1. Antibody

| | |
|---|---|
| Name: | Catumaxomab |
| Characteristic features: | bispecific trifunctional antibody (anti-EpCAM x anti-CD3) |
| Supplier/Manufacturer: | Lindis Biotech GmbH/Trion Pharma GmbH |
| Batch number: | 1Q01-0/1 |
| Concentration: | 100 µg/ml |
| Storage conditions: | 2-8°C |

### 1.2. Intraoperative blood

### 1.2.1 MAT/ Reservoir

Blood and purge solvent accumulated during surgery was collected in a reservoir containing a bone splinter filter (MAT/Reservoir sample).

### 1.2.2 EK b (EC)

The blood and purge solvent mixture collected in the reservoir was centrifuged and washed using a Cell Saver machine (Fresenius), resulting in an erythrocyte concentrate (EC) (Erythrozytenkonzentrat: EK_b sample).

### 1.2.3 LDF

The erythrocyte concentrate (EC or EK_b) was filtered using a 40 µm leukocyte depletion filter (LDF, Haemonetics), resulting in an erythrocyte concentrate containing almost no white blood cells anymore (LDF sample).

All samples for analysis were extracted via an output connection/ an outlet and collected in sterile tubes.

### 2. Methods

### 2.1 Sample generation by University hospital Frankfurt

All samples were collected during surgery as described in 1.1.2 and the blood samples for the tumor cell detection were sent immediately after the surgery via TNT at room temperature, arriving at Trion Research GmbH next morning before 9 a.m. The samples collected for antibody analysis were frozen within 1-2h (-20°C) and sent to Trion Research GmbH on dry ice. The blood samples for cytokine analysis were centrifuged, the supernatant (plasma) collected, frozen within 1-2h (-20°C) and also sent to Trion Research GmbH on dry ice.

### 2.2 Methods carried out by Trion Research GmbH

In a first step the peripheral blood mononuclear cells (PBMC) and possible tumor cells from samples deriving from MAT, EK_b and LDF were isolated by ficoll density centrifugation. In a second step, cytospins were generated. Performances to process the blood samples and to generate cytospins were done according to the following SOPs:
- AY-028.01 "Isolierung von PBMC"
- TR-AS-0107-V01 "Determination of cell count and cell viability"
- TR-SOP-KF-004 "Preparation of Cytospins in clinical studies with Catumaxomab"
Additionally, the antibody concentration in the MAT, EK_b and LDF samples was determined via ELISA measurement, applying the following SOP:
- AY-006.02 Catumaxomab-ELISA

No de-lipidation of samples was performed which is considered not critical as samples were not fatty. EK_b and LDF samples were not centrifuged (in contrast to MAT samples) but mixed 1:1 with sample dilution buffer and directly applied to coated ELISA plates. This procedure achieves more accurate results as shown in a preliminary spiking study. All samples for antibody measurement were collected during performance of Catuvab procedure at study side in Frankfurt, frozen within 1-2 hours and stored at <-15°C until batch-wise shipment on dry ice to Trion Research. There, samples were stored at <-15°C until measurement.

### 2.3 Methods performed by a external certified laboratory

An external certified laboratory analysed the cytospins for the presence of EpCAM-positive tumor cells using the antibody BER-EP4.

### 2.4 Methods performed by Synlab MVZ Labor München Zentrum GbR

The Synlab MVZ Labor measured the cytokine profiles in the MAT, EK_b and LDF samples.

### 3 Results

The Catuvab procedure was applied extra-corporal to the intraoperative blood of 15 patients during surgery. To the collected blood of one patient (No. 9) no antibody was applied (no antibody detectable in reservoir, data not shown), so this patient and related samples were taken out of statistical calculations. Only tumor indications were selected, known to be EpCAM positive according to literature (see table 1). The volume of the intraoperative blood mixture (blood and dilution fluid) ranged from 500ml up to 2800ml and the volume of the added dilutive solution during surgery from 0 up to 2500ml. The volume of undiluted intraoperative blood ranged between 300 and 1300ml for the group of patient samples treated with 2.5µg Catumaxomab to generate tumor cell aggregates. For the group of patient samples treated with 5µg, the volume of undiluted (and diluted) intraoperative blood ranged from 300ml (1400ml) up to 2550ml (2600ml).

### 3.1 Detection of EpCAM positive tumor cells in intraoperative blood

In ten out of 16 intraoperative patient blood samples (62.5%), EpCAM positive tumor cells were detected within the different Catuvab procedure steps (reservoir and/or EC). The amount of EpCAM positive tumor cells ranged from 69, found in an EC sample (before filtration) to 263.076 in the MAT- reservoir. Importantly, no tumor cells were found after the last purification step (filtration) within the EC final product (table 2). The staining of tumor cells was performed by the team of an external certified laboratory which has a long experience for the detection of EpCAM positive tumor cells derived from carcinomas.

### 3.2 Measurement of proinflammatory cytokines IL-6 and IL-8

During the Catuvab procedure also samples were taken to measure proinflammatory cytokines to assess safety parameters. Thus, IL-6 and IL-8 were measured in the reservoir before addition of the crosslinking antibody Catumaxomab as well as in the EC before and after filtration (final product). As Catumaxomab is well known to activate different types of immune cells, the aim of the measurements was to determine a potential increase of proinflammatory cytokines during the Catuvab procedure.

As shown in table 1 the values for IL-6 (and ***IL-8 in bold)*** in intraoperative blood ranged from below level of quantification (BLQ of 4pg/ml; ***6pg*/*ml***) up to 2633pg/ml; ***518pg*/*ml*** (mean: 662pg/ml; ***129pg*/*ml*)** in the reservoir before addition of Catumaxomab. After addition of Catumaxomab the values ranged from 49 up to 1070 pg/ml (mean: 339pg/ml; ***323pg*/*ml***) in the EC (before filtration). In the EC after filtration values ranged from 20; ***BLQ*** up to 2488pg/ml; ***139pg*/*ml*** (mean: 516pg/ml; ***46pg*/*ml***)**.** As during the Catuvab procedure the volumes of intraoperative blood decreased from e.g. 2800ml in the reservoir (mean: 1461 ml) to 40ml in EC (mean: 99ml) also total amounts of cytokines were calculated in the reservoir and ECs (after filtration) to assess (i) the reduction of total amounts of cytokines during the Catuvab procedure and (ii) the amounts of cytokines which would be potentially reinfused in the patient. The total amounts of cytokine IL-6; ***IL-8*** ranged from 5.2ng, ***8.4ng*** up to 7372ng; ***901ng*** (mean: 1502ng; ***204ng***) in the collected intraoperative blood in the reservoir. After completion of the Catuvab procedure the values of IL-6; ***IL-8*** ranged from 1 ng; ***0.1ng*** up to 264ng; ***16ng*** (mean: 53ng; ***3.9ng*)** in the EC (final product). Regarding the mean values a 28- ; ***52*** -fold reduction was shown for the total amounts of IL-6; ***IL-8*** in the final product (EC) compared to the unprocessed collected intraoperative blood in the reservoir.

### 3.3 Assessment of residual antibody (Catumaxomab) in the EC final product after filtration

An important prerequisite for a medical device is the absence of antibody or very low residual antibody in the final product, which is intended to be reinfused to the patient. Therefore, a very sensitive ELISA-assay with a quantification limit of 125pg/ml was used to measure residual antibody.

During the pilot study different ways to apply the antibody were investigated. Thus, for the patients 1-10, the way of antibody application was not clearly defined and in some cases the antibody was applied first in the reservoir before the collection of intraoperative blood was started. Antibody measurements revealed that such an application schedule led to an unsatisfactory distribution of antibody, as local high concentration of antibody occurred at the bottom of the reservoir. As a result, unbound antibody accumulated and was detected in relatively high amounts still at the end of the Catuvab procedure in the final EC product. This was the case for patients 2 and 7 where relatively high antibody concentrations were measured of 1.694pg/ml and 1.026pg/ml after application of 2.5µg antibody (table 3).

In so far, to improve the situation, the antibody was applied in patients 11-16 only after a minimum collected intraoperative blood volume of 350ml in the reservoir to allow a better interaction and binding of the antibody with immune cells and EpCAM positive tumor cells. This change in the experimental setting obviously improved the situation, as in patients 11 and 16 the antibody concentration dropped to BLQ and in patient 15 to 160pg/ml after application of 2.5µg.
Another important finding was, that in case of higher volumes of intraoperative blood (>1500ml), when additional antibody (+2.5µg) was applied to the reservoir, also higher concentrations of antibody could be found in the final product. Thus, in patients 12 and 13 also relatively high antibody concentrations with 888pg/ml and 628pg/ml were detected.

Therefore, it was decided for the protocol of the main study, that after collection of 1500ml intraoperative blood and supply with 2.5µg antibody, centrifugation has to be started first, before a new collection of intraoperative blood with initiation of a new Catuvab procedure and supply with new antibody (2.5µg) can be started.

Another important aim of this study is the measurement of the total amount of antibody, which could be potentially applied together with the final EC back to the patient.

Here, the total amounts of detected antibody ranged from BLQ up to 69ng (2.5µg application group) in patients 1-10 before the antibody application was improved. After establishment of the improved antibody application in the reservoir, the total amounts of antibody found in the final EC ranged from BLQ up to 9ng in the 2.5µg application group and BLQ up to 59ng in the 5µg group (table 3).

### 4 Discussion

In the above Example wherein the intraoperative blood of 15 tumor patients were subjected to a medical device procedure containing a trifunctional antibody combined with centrifugation and filtration steps, parameters were optimized to fulfill the following criteria:
1) Removal of tumor cells from the produced erythrocyte concentrate out of intraoperative blood
2) Reduction of proinflammatory cytokines produced in the operational field by traumata through surgery
3) Reduction of trifunctional antibody utilized to crosslink immune- and tumor- cells to uncritical amount in the final erythrocyte concentrate (EC) ready for reinfusion.

For optimization, the following parameters were investigated and defined:
1) **Minimum amount of lymphocytes and monocytes** with sufficient binding sites for the utilized trifunctional antibody with specificities anti-EpCAM, anti-CD3 and a Fc-part binding to Fc-gamma receptor positive cells. The minimum amount of immune cells would allow a quantitative binding of utilized trifunctional antibodies which could also crosslink with the anti-EpCAM binding arm tumor cells of epithelial origin to generate cell aggregates (cell complexes).
2) Thus, experiments demonstrate that an amount of **2.5µg said anti-EpCAM x anti-CD3** trifunctional **antibody** could be quantitatively bound by a **minimum of 300ml undiluted intraoperative blood containing about 300x10e6 lymphocytes and monocytes.** The minimum amount of undiluted intraoperative blood could be diluted up to five-fold with e.g. solo-sterofundin solution still fulfilling the above mentioned criteria. As a result, residual antibody was determined as below limit of quantification (BLQ) up to 9ng in the final EC product.
3) Crosslinking of immune cells with tumor cells by a trifunctional antibody followed by centrifugation within a MAT-cell saver machine produced an erythrocyte concentrate **strongly reduced by immune cells** and volume. Subsequent filtration of the EC generated a final EC product ready for reinfusion with a **28- and 52-fold reduced amount of proinflammatory cytokines IL-6 and IL-8,** respectively, compared with unprocessed operational blood.

Detailed analysis of the patients with substantial residual antibody in EC after LDF in the proof-of concept study revealed circumstances responsible for this observation, which have to be avoided in above example. Thus, a minimal volume (300-400 ml) of intraoperative blood must be collected in the IBS-reservoir **before** application of the antibody in the reservoir to avoid local over-concentration of antibody. Secondly, a minimum of undiluted intraoperative blood (400 ml) can be collected before starting the Catuvab procedure (start of centrifugation to produce EC) to provide sufficient binding sites on lymphocytes for the antibody. In the case that a maximum volume of 1500 ml collected intraoperative blood mixture (blood and diluent) is reached in the reservoir, a first centrifugation round of the IBS device can be started with the first antibody dose of 2.5µg before, in case of further intraoperative blood collected from the surgical field, a further 2.5µg dose of catumaxomab can be added to the reservoir.

If more than 1500 ml of intraoperative blood is available from an individual patient, a second round of IBS can be performed if criteria, like for the first run are fulfilled. Additional runs (three or more) are not permitted to limit the amount of residual Ab being re-infused. Generally, a total amount of residual antibody accumulated during two runs in the ECs should not be more than 70ng.

The third important parameter especially in light of an approval of the Catuvab procedure as medical device is the amount of residual antibody in the final EC product. Here, two changes [(i) a minimum amount of intraoperative blood has to be collected before antibody is added, (ii) in case that 1500ml of intraoperative blood is collected the procedure has to be started and for higher volumes of intraoperative blood a second procedure has to be initiated] of the procedure were introduced during the study (described in the results section) which led to strongly reduced residual antibody in the final product. Thus, after implementation of the changes, only total antibody amounts between the quantification limit (125pg/ml) and 9ng were found in the final EC product.

**Table 1**

| | **Patient number** | **1** | **2** | **3** | **4** | **5** | **6** | **7** | **8** | **9** | **10** | **11** | **12** | **13** | **14** | **15** | **16** |
|---|---|---|---|---|---|---|---|---|---|---|---|---|---|---|---|---|---|
| | **Indication of cancer** | **Ösophag.** | **Cholangio.** | **Rectal** | **Ösoph.** | **Cholang.** | **Pancreas** | **Gist** | **Colon** | **Cholang.** | **Coecum + Ovar** | **Panc.** | **Pancr.** | **Gallen-gang** | **Ractal** | **Gallen-gang** | **Perihiläres CCC** |
| | **OP blood [ml]** | **1400** | **500** | **2800** | **1600** | **1100** | **1600** | **800** | **950** | **1000** | **2200** | **800** | **1700** | **1661** | **2600** | **1400** | **800** |
| | **Dilution fluid [ml]** | **550** | **0** | **2500** | **1000** | **500** | **500** | **300** | **300** | **700** | **200+800** | **150** | **1400** | **900** | **50** | **100** | **200** |
| Cytokines | Reservoir | BLQ/6 | BLQ/92 | 2633/322 | 90/16 | 257/518 | 121/25 | 163/75 | 23/17 | BLQ/9 | 2617/76 | 688/232 | 43/108 | 284/100 | 2271/157 | 76/56 | 244/114 |
| **IL-6/IL8** | EC | 1054/1032 | 25/103 | 219/1046 | 727/569 | 221/199 | 91/25 | 267/102 | 17/10 | - | 1070/383 | 49/353 | 243/97 | 317/138 | 191/180 | 264/284 | 29/821 |
| pg/ml | LDF | 408/24 | 55/112 | 236/139 | 780/13 | 317/58 | 114/9 | 260/24 | 22/BLQ | BLQ/BLQ | 2488/72 | 20/5 | 492/86 | 184/17 | 554/11 | 1289/67 | BLQ/21 |
| Cytokines IL-6/IL-8 | Reservoir | 14.7/8.4 | 5.2/46 | 7372/901 | 144/26 | 283/570 | 194/40 | 130/60 | 22/16 | 11/09 | 5757/167 | 550/186 | 73/184 | 472/166 | 5904/408 | 106/78 | 195/91 |
| Total (ng) | LDF | 43.6/2.6 | 2.3/4.6 | 27/16 | 49/0.8 | 20/4 | 17/1 | 17/2 | 1/0.1 | 0.4/0.1 | 264/8 | 1/0.2 | 33/6 | 08/01 | 177/4 | 77/4 | 2/4 |

| | | | | | | | | | | | | | | | | | |
|---|---|---|---|---|---|---|---|---|---|---|---|---|---|---|---|---|---|
| Table 1: Results of measurement of cytokines IL-6 and IL-8 BLQ = 9.8 pg/ml for IL-6 and 3.1 pg/ml for IL-8 | | | | | | | | | | | | | | | | | |

**Table 2**

| Patient number | **1** | **2** | **3** | **4** | **5** | **6** | **7** | **8** | **9** | **10** | **11** | **12** | **13** | **14** | **15** | **16** |
|---|---|---|---|---|---|---|---|---|---|---|---|---|---|---|---|---|
| **Indication of cancer** | **Ösophag.** | **Cholangio.** | **Rectal** | **Ösoph.** | **Cholang.** | **Pancreas** | **Gist** | **Colon** | **Cholang.** | **Coecum + Ovar** | **Panc.** | **Pancr.** | **Gallen-gang** | **Rectal** | **Gallen-gang** | **Perihiläres CCC** |
| **OP blood [ml]** | **1400** | **500** | **2800** | **1600** | **1100** | **1600** | **800** | **950** | **1000** | **2200** | **800** | **1700** | **1661** | **2600** | **1400** | **800** |
| **Dilution fluid[ml]** | **550** | **0** | **2500** | **1000** | **500** | **500** | **300** | **300** | **700** | **200+800** | **150** | **1400** | **900** | **50** | **100** | **200** |
| total number of tumor cells in MAT/Reservoir | 7.600 | 0 | 263.076 | 0 | 14.322 | 108.984 | 108 | 0 | 0 | 101.525 | 15.529 | 0 | 0 | 0 | 0 | 0 |
| total number of tumor cells in EC/ EK_b | 0 | 192 | 6.835 | 0 | 2.756 | 0 | 0 | 0 | 0 | 4.163 | 1.886 | 69 | 0 | 0 | 419 | 0 |
| total number of tumαr cells in EC after filtration (LDF) | 0 | 0 | 0 | 0 | 0 | 0 | 0 | 0 | 0 | 0 | 0 | 0 | 0 | 0 | 0 | 0 |

| | | | | | | | | | | | | | | | | |
|---|---|---|---|---|---|---|---|---|---|---|---|---|---|---|---|---|
| Table 2: Results of measurement of EpCAM-positive tumor cells Tumor cell numbers in the reservoir and erythrocyte concentrates (EC) were assessed in test samples and calculated to the total volume. In EC after filtration the total volume was consumed for tumor cell assessment | | | | | | | | | | | | | | | | |

**Table 3**

| **Patient number** | **1** | **2** | **3** | **4** | **5** | **6** | **7** | **8** | **9** | **10** | **11** | **12** | **13** | **14** | **15** | **16** |
|---|---|---|---|---|---|---|---|---|---|---|---|---|---|---|---|---|
| **Indication of cancer** | **Ösophag.** | **Cholangio.** | **Rectal** | **Ösoph.** | **Cholang.** | **Pancreas** | **Gist** | **Colon** | **Cholang.** | **Coecum + Ovar** | **Panc.** | **Pancr.** | **Gallen-gang** | **Rectal** | **Gallen-gang** | **Perihiläres ccc** |
| **OP Mood [ml]** | **1400** | **500** | **2800** | **1600** | **1100** | **1600** | **800** | **950** | **1000** | **2200** | **800** | **1700** | **1661** | **2600** | **1400** | **800** |
| **Dilution fluid [ml]** | **550** | **0** | **2500** | **1000** | **500** | **500** | **300** | **300** | **700** | **200+800** | **150** | **1400** | **900** | **50** | **100** | **200** |
| **EC after LDF, residual Ab[pg/ml]** | BLQ | 1.694 | BLQ | BLQ | 205,0 | BLQ | 1.026 | 491,0 | No Ab detectable | 252,0 | BLQ | 888,0 | 628,0 | BLQ | 160,0 | BLQ |
| **Ab total [ng]** | | 69,0 | | | 13,0 | | 68,0 | 22,0 | | 27,0 | | 59,0 | 26,0 | | 9,0 | |
| **Vol. EC [ml]** | 142,0 | 76,0 | 150,0 | 98,0 | 97,0 | 187,0 | 101,0 | 80,0 | 70,0 | 141,0 | 75,0 | 101,0 | 77,0 | 373,0 | 133,0 | - |
| **Vol EC after LDF [ml]** | 107,0 | 41,0 | 115,0 | 63,0 | 62,0 | 152,0 | 66,0 | 45,0 | 35,0 | 106,0 | 40,0 | 66,0 | 42,0 | 320,0 | 60,0 | 200,0 |
| **amount of added antibody applied [µg]** | 5,0 | 2,5 | 2,5 | 2,5 | 2,5 | 5,0 | 2,5 | 2,5 | (-) | 5,0 | 2,5 | 5,0 | 5,0 | 5,0 | 2,5 | 2,5 |

| | | | | | | | | | | | | | | | | |
|---|---|---|---|---|---|---|---|---|---|---|---|---|---|---|---|---|
| BLQ: below limit of quantification, 125pg/ml Table 3: Results of antibody measurement | | | | | | | | | | | | | | | | |

## Claims

1. An *ex vivo* method for removal of tumor cells from intra-operatively salvaged blood comprising the following steps:
(i) collecting an intra-operatively salvaged blood which may contain immune cells and tumor cells in a reservoir;
(ii) contacting said intra-operatively salvaged blood from step (i) in the reservoir with at least one trifunctional antibody and/or with a scaffold protein, wherein said antibody or said scaffold protein comprises the following properties:
a) binding to a T cell;
b) binding to a tumor-associated antigen on a tumor cell;
c) binding via its Fc-portion to an Fc-receptor positive cell,
to obtain an intra-operatively salvaged blood containing cell aggregates, wherein said cell aggregates comprise said trifunctional bispecific antibody and/or said scaffold protein;
(iii) separating an erythrocyte concentrate from the intra-operatively salvaged blood obtained from step (ii) via centrifugation;
(iv) washing said erythrocyte concentrate from step (iii);
(v) filtering the erythrocyte concentrate from step (iv) to remove residual of said aggregates and/or residual of said cell-bound trifunctional bispecific antibody and/or scaffold protein.

2. The ex vivo method according to claim 1, wherein said trifunctional antibody is selected from the group consisting of a bispecific, trispecific, tetraspecific and multispecific antibody, preferably a whole IgG bispecific antibody

3. The *ex vivo* method according to claim 1 or 2, wherein the amount of said at least one trifunctional antibody and/or said scaffold protein used to contact with said intra-operatively salvaged blood in step (ii) is 2.5 µg or more, preferably 2.5 µg or more and 5.0 µg or less.

4. The *ex vivo* method according to any one of claims 1 to 3, wherein said intra-operatively salvaged blood in step (i) has a volume of 300 ml or more, preferably 400 ml or more collected in the reservoir before application of the trifunctional bispecific antibody, optionally the method further comprises a step of diluting the intra-operatively salvaged blood in step (i) to a volume of 350ml to 2800 ml, preferably 350 ml to 2000 ml, which contains at least 300ml - 500ml undiluted intraoperatively salvaged blood.

5. The *ex vivo* method according to any one of claims 1 to 4, wherein the method comprises at least one further round of steps (i) to (v), and wherein at least in the first round the intra-operatively salvaged blood for contacting with said trifunctional antibody is in a volume of 400-1500 ml.

6. The *ex vivo* method according to claim 5, wherein the intra-operatively salvaged blood is a mixture of blood and dilution.

7. The *ex vivo* method according to any one of claims 1 to 6, wherein in step (ii) said at least one trifunctional antibody and/or said scaffold protein is contacted with said intra-operatively salvaged blood for a time period of 10 - 180 minutes to obtain the intra-operatively salvaged blood containing cell aggregates, preferably 20-90 minutes, more preferably 30-60 minutes, and optionally at a temperature of 19-25°C, preferably at room temperature.

8. The *ex vivo* method according to any one of claims 1 to 7, wherein said cell aggregates comprise antibodies, tumor cells and immune cells, wherein the immune cells are preferably T cells and/or Fc-gamma receptor positive cells.

9. The *ex vivo* method according to any one of claims 1 to 8, wherein a filter is used in step (v) for filtering the erythrocyte concentrate from step (iv), and wherein said filter is preferably a leukocyte depletion filter.

10. The *ex vivo* method according to any one of claims 1 to 9, wherein said at least one trifunctional antibody is selected of a group of antibodies with the following isotype combinations:
rat-IgG2b/mouse-IgG2a,
rat-IgG2b/mouse-IgG2b,
rat-IgG2b/human-IgG1,
mouse-[VH-CH1; VL-CL]-human-IgG1/rat-[VH-CH1, VL-CL]-human-IgG1-[hinge]-human-IgG3*-[CH2-CH3]
[* = Caucasian allotypes G3m(b+g) = no binding to protein A].

11. The *ex vivo* method according to any one of claims 1 to 10, wherein said tumor associated antigen is selected from the group consisting of: EpCAM, Her2neu, EGFR, CD30, CD20, CD22, MUC1, MUC1* with changed glycosylation pattern, PSMA, CD33, MCSP, cMet, EphA2, Endosialin, Carboanhydrase IX, IGF-1R, FAP-alpha, CD19, GD2, CEA, FR, proteoglycans, G250, GC182, GT468, GT512, preferably the tumor associated antigen is EpCAM.

12. The *ex vivo* method according to any one of claims 1 to 11, wherein said trifunctional antibody and/or said scaffold protein binds to the T cell through a T cell surface antigen, and wherein the T cell surface antigen is selected from a group consisting of CD2, CD3, CD4, CD8, CD28, CD40L and CD44, preferably the T cell surface antigen is CD3.

13. The *ex vivo* method according to any one of claims 1 to 12, wherein said at least one trifunctional antibody and/or said scaffold protein comprises a binding site in its Fc-portion for Fcγ receptor type I, II and/or III.

14. The *ex vivo* method according to any one of claims 1 to 13, wherein said at least one trifunctional antibody and/or said scaffold protein is capable of binding monocytes, macrophages, dendritic cells, natural killer cells and/or activated neutrophils by their Fcγ receptor type I, II and/or III.

15. A trifunctional antibody which comprises the following properties:
a) binding to a T cell;
b) binding to a tumor-associated antigen on a tumor cell;
c) binding via its Fc-portion to an Fc-receptor positive cell,
for use in a method of treating tumor or cancer, comprising (i) collecting an intra-operatively salvaged blood which may contain immune cells and tumor cells;
(ii) contacting said intra-operatively salvaged blood from step (i) with said trifunctional antibody to obtain an intra-operatively salvaged blood containing cell aggregates, wherein said cell aggregates comprise said trifunctional antibody;
(iii) separating an erythrocyte concentrate from the intra-operatively salvaged blood obtained from step (ii) via centrifugation, preferably by density gradient centrifugation;
(iv) washing said erythrocyte concentrate from step (iii);
(v) filtering the erythrocyte concentrate from step (iv) to remove residual of said aggregates and/or residual of said cell-bound trifunctional antibody.
